(19) Europäisches Patentamt
European Patent Office
Office européen des brevets

(11) Numéro de publication: **0 102 305**
**B1**

(12) **FASCICULE DE BREVET EUROPEEN**

(45) Date de publication du fascicule du brevet: **03.07.85**

(51) Int. Cl.⁴: **C 07 C 85/24**, C 07 C 87/60

(21) Numéro de dépôt: **83420139.4**

(22) Date de dépôt: **11.08.83**

(54) **Procédé de préparation d'anilines métachlorées.**

(30) Priorité: **24.08.82 FR 8214682**

(43) Date de publication de la demande:
**07.03.84 Bulletin 84/10**

(45) Mention de la délivrance du brevet:
**03.07.85 Bulletin 85/27**

(84) Etats contractants désignés:
**AT BE CH DE FR GB IT LI LU NL SE**

(56) Documents cités:
**EP-A-0 015 219**
**EP-A-0 051 782**

**J. ORG. CHEM., vol. 22, septembre 1957, Washington D.C., (US), A.H. BLATT et al.: "Replacement of halogen by hydrogen in nitro aryl halides", pages 1046-1049**
**AUST. J. CHEM., vol. 26, 1973, D.J. BROWN et al.: "Pyrimidine reactions. XXIV. The dehalogenation of 2-halogenopyrimidines by hydriodoc acid", pages 443-447**

(73) Titulaire: **RHONE-POULENC AGROCHIMIE, 14-20, rue Pierre Baizet, F-69009 Lyon (FR)**

(72) Inventeur: **Cordier, Georges, 50 Chemin des Hermières, F-69340 Francheville (FR)**

(74) Mandataire: **Chrétien, François et al, RHONE-POULENC AGROCHIMIE BP 9163, F-69263 Lyon Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

EP 0 102 305 B1

## Description

La présente invention se rapporte à un procédé de préparation d'anilines substituées par le chlore en position méta par déchloration d'amines aromatiques plus fortement chlorées. Ces anilines métachlorées sont des intermédiaires notamment pour la fabrication de matières actives agrochimiques.

On a déjà proposé de nombreux procédés de préparation d'anilines chlorées en position méta par hydrodéchloration de polychloranilines avec de l'hydrogène sous pression en milieu liquide en présence d'un acide fort et d'un catalyseur à base de métal noble. Ces procédés nécessitent des pressions élevées qui, associées à des températures relativement élevées et aux conditions acides, obligent à travailler en autoclave avec de graves problèmes de corrosion. On a tenté de modérer la température et la pression en opérant en présence de dérivés à base d'iode mais on a toujours recours à des catalyseurs hétérogènes métalliques.

La présente invention a pour but de préparer les chloroanilines substituées en méta par élimination sélective d'atomes de chlore d'une manière encore plus simple donc plus facilement industrialisable.

L'invention concerne plus particulièrement un procédé de préparation d'anilines substituées en position méta par du chlore de formule générale

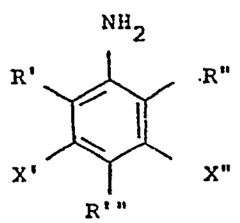

(B)

dans laquelle:

X' et X", identiques ou différents représentent chacun un atome de chlore, un radical alcoyle contenant de 1 à 4 atomes de carbone, un radical alcoxyle contenant de 1 à 4 atomes de carbone, au moins l'un des X' et X" étant obligatoirement un atome de chlore, l'un des X' et X" pouvant de plus être un atome d'hydrogène,

– R', R" et R‴, identiques ou différents, représentent chacun un atome de chlore, un radical alcoyle contenant de 1 à 4 atomes de carbone, un radical alcoxyle contenant de 1 à 4 atomes de carbone ou un radical phényle, benzyle ou phénoxyle, le noyau phényle de ces radicaux pouvant être substitué par au moins un atome d'halogène, notamment de chlore, ou un radical alcoyle contenant de 1 à 4 atomes de carbone ou alcoxyle de 1 à 4 atomes de carbone, au plus deux d'entre eux pouvant être un atome d'hydrogène, par réaction d'une aniline polychlorée de formule générale:

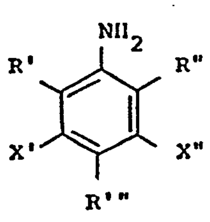

(A)

dans laquelle X', X", R', R", R‴ ont les mêmes significations que précédemment avec la condition supplémentaire que l'un au moins des R', R" et R‴ représente un atome de chlore, en phase liquide, avec de l'iodure d'hydrogène, selon le schéma:

$$A + 2n\ HI \rightarrow B + n\ I_2 + n\ HCl$$

dans lequel n est un nombre entier de 1 à 3 représentant le nombre d'atomes de chlore à enlever par mole de l'aniline polychlorée A.

La quantité d'iodure d'hydrogène qu'il faut engager dans la réaction est au moins stœchiométrique et de préférence en excès, c'est-à-dire que le rapport molaire $\frac{HI}{n}$ est d'une manière générale compris entre 2/1 et 20/1 et de préférence entre 2/1 et 10/1.

La réaction est effectuée soit en milieu aqueux, auquel cas l'iodure d'hydrogène est engagé sous forme d'une solution d'acide iodhydrique, soit en milieu organique dans un solvant organique inerte dans les conditions de la réaction, et notamment vis-à-vis de l'iodure d'hydrogène engagé et de l'iode produit ainsi que du chlorure d'hydrogène, comme par exemple un solvant aromatique comme le toluène ou le xylène, un solvant aromatique chloré tel qu'un chlorobenzène ou un solvant aliphatique chloré.

On peut de manière avantageuse, pour accélérer la réaction, ajouter au milieu réactionnel un acide fort soit minéral comme l'acide chlorhydrique ou l'acide sulfurique lorsqu'on opère en milieu aqueux, soit organique comme l'acide acétique, l'acide trichloroacétique, l'acide trifluoroacétique, l'acide méthanesulfonique ou para-toluène-sulfonique, lorsqu'on opère en milieu solvant organique.

Bien entendu l'iodure d'hydrogène peut être soit engagé tel quel, soit formé in situ dans les conditions de la réaction comme par exemple un mélange d'iodure alcalin et d'acide chlorhydrique.

Par ailleurs, en milieu organique, la réaction dégageant du chlorure d'hydrogène, la pression de celui-ci croît: il n'y a pas d'inconvénient à travailler sous pression autogène mais on préfère industriellement limiter celle-ci à au plus 100 bars et de préférence à au plus 50 bars. Pour cela on opère sous courant de HI avec élimination continue de l'HCl formé.

La température de réaction est généralement comprise entre 90 et 250°C et de préférence de 110 à 220°C. A des températures plus basses, la réaction est lente et de plus les iodhydrates d'amines ne sont pas complètement solubles dans le milieu. A partir de 100°C, le milieu est homogène et la réaction s'effectue avec un rendement pratiquement quantitatif, l'élévation de température ayant un effet d'accélération de la réaction. Par ailleurs, il n'y a plus d'intérêt industriel à travailler au-delà de 250°C.

Comme composés de formule A pouvant servir pour la mise en œuvre du procédé, on peut citer notamment ceux dans la formule desquels R', R" et R‴ représentent un atome d'hydrogène ou de

chlore, c'est-à-dire les tétrachloro-2,3,4,5, -2,3,4,6 et -2,3,5,6 anilines ainsi que la pentachloroaniline, la préférence étant donnée aux dichloro-3,4 et 2,3, à la trichloro-3,4,5 et aux tétrachloro-2,3,4,5 et -2,3,5,6 anilines.

Parmi les anilines chlorées en méta de formule B pouvant être préparées par le procédé selon l'invention, on peut citer notamment celles dans la formule desquelles R', R'' et R''' représentent un atome d'hydrogène ou de chlore, c'est-à-dire les dichloro-2,3 -2,5 et -3,4 anilines, les trichloro-2,3,4 -2,3,5, -2,3,6, -2,4,5 et -3,4,5 anilines et de préférence la chloro-3 aniline et la dichloro-3,5 aniline. On peut obtenir ces anilines chlorées soit seules, soit en mélanges selon la composition de départ et le degré de transformation.

Le procédé selon l'invention peut être effectué en continu ou en discontinu. En fin de réaction, l'aniline métachlorée obtenue peut être séparée par tout moyen connu en soi, par exemple extraction à l'aide d'un solvant et/ou par distillation.

Le procédé selon l'invention permet d'obtenir de manière sélective des anilines métachlorées dans des conditions particulièrement avantageuses pour sa mise en œuvre industrielle puisqu'on opère en milieu complètement homogène, sans catalyseur et à des pressions très modérées.

Les exemples suivants sont donnés à titre indicatif mais non limitatif pour illustrer l'invention. Les taux de transformation et les rendements sont des rapports molaires.

*Exemple 1:*

Dans un réacteur de 200 $cm^3$ de capacité, revêtu intérieurement de tantale et résistant à la pression, on charge:

– 0,1 mol de tétrachloro-2,3,4,5 aniline,

– 50 ml d'une solution aqueuse à 8 mol/l d'acide iodhydrique.

On chauffe pendant 16 h à 135°C, sous agitation et sous pression autogène.

Après ce temps de réaction, on ajoute au milieu réactionnel refroidi de l'eau et du toluène (ou du monochlorobenzène) en quantité suffisante pour obtenir deux phases liquides claires. Celles-ci sont séparées par décantation, la phase aqueuse est débarrassée de l'iode par une série d'extractions au toluène (ou au monochlorobenzène). La phase organique est distillée sous pression atmosphérique, ce qui permet de séparer la presque totalité de l'iode des polychloranilines.

Le résidu organique est analysé par chromatographie en phase vapeur.

Dans ces conditions, on observe que le taux de transformation (TT) de la tétrachloro-2,3,4,5 aniline est de 99,5%, le rendement réel en dichloro-3,5 aniline est de 61,1%; il reste 37,5% de trichloro-2,3,5 aniline intermédiairement formée.

Si l'on répète cette réaction en chauffant pendant 32 h, le rendement réel en dichloro-3,5 aniline est quantitatif.

*Exemples 2 à 7:*

On opère comme à l'exemple 1, en faisant varier la concentration en tétrachloroaniline (2,3,4,5 TTCA), celle en acide iodhydrique (HI), la température (T), la durée avec, dans les deux cas, addition d'acide chlorhydrique (HCl), comme acide fort.

Le tableau ci-dessous donne les valeurs des paramètres qui varient ainsi que le taux de transformation de la tétrachloro-2,3,4,5 aniline et le rendement réel en dichloro-3,5 aniline (3,5 DCA) et en son intermédiaire trichloro-2,3,5 aniline (2,3,5 TCA). A noter que, dans cette réaction, n est égal à 2.

*Tableau I*

| Exemple N° | [2,3,4,5 TTCA] (mol/l) | [HI] (mol/l) | $\frac{[HI]}{n}$ | [HCl] (mol/l) | T (°C) | Durée (h) | T T (%) TTCA | R R (%) 3,5 DCA | R R (%) 2,3,5 DCA |
|---|---|---|---|---|---|---|---|---|---|
| 2 | 2 | 8 | 2 | — | 135 | 16 | 99,5 | 61,1 | 37,5 |
| 3 | 1 | 4 | 2 | — | 135 | 5 | 97,6 | 19,1 | 78,5 |
| 4 | 1 | 8 | 4 | — | 135 | 5 | 100,0 | 91,6 | 7,4 |
| 5 | 1 | 4 | 2 | 6 | 135 | 5 | 99,5 | 56,5 | 43,4 |
| 6 | 2 | 8 | 2 | — | 163 | 1 | 98,0 | 57,2 | 36,5 |
| 7 | 0,5 | 2,4 | 2,4 | 7,6 | 163 | 1 | 100,0 | 81,8 | 13,6 |

Si l'on répète ces essais en chauffant deux fois plus longtemps, on obtient un rendement réel quantitatif en dichloro-3,5 aniline.

*Exemple 8:*

On opère comme à l'exemple 1, en remplaçant la tétrachloro-2,3,4,5 aniline par 0,05 mol de tétrachloro-2,3,5,6 aniline et en chauffant pendant 5 h. On constate que, dans ces conditions, le taux de transformation de la tétrachloro-2,3,5,6 aniline est de 96%, le rendement réel en dichloro-3,5 aniline est de 81,2% et il reste 14,7% de trichloro-2,3,5 aniline intermédiaire non transformée.

En opérant comme précédemment mais avec une durée de chauffage double, le taux de transformation de la tétrachloroaniline est de 100% et le rendement réel en dichloro-3,5 aniline est quantitatif.

*Exemples 9 à 15:*

On opère comme aux exemples 2 à 7 en remplaçant la tétrachloro-2,3,4,5 aniline par la trichloro-3,4,5 aniline, auquel cas n est égal à 1.

Le tableau ci-dessous donne les valeurs des paramètres qui varient ainsi que le rendement réel en dichloro-3,5 aniline.

*Tableau II*

| Exemple N° | [3,4,5 TCA] (mol/l) | [HI] (mol/l) | [HI]/n | [HCl] (mol/l) | T (°C) | Durée (h) | TT (%) A | RR (%) 3,,5 DCA |
|---|---|---|---|---|---|---|---|---|
| 9 | 2 | 4 | 2 | — | 135 | 16 | 70,5 | 70,5 |
| 10 | 2 | 6 | 3 | — | 135 | 5 | 90,3 | 90,3 |
| 11 | 2 | 8 | 4 | — | 135 | 5 | 98,0 | 98,0 |
| 12 | 2 | 8 | 4 | — | 135 | 2 | 93,5 | 93,5 |
| 13 | 2 | 4 | 2 | — | 163 | 1 | 69,5 | 69,5 |
| 14 | 2 | 4 | 2 | 4 | 163 | 1 | 78,0 | 78,0 |
| 15 | 2 | 8 | 4 | — | 105 | 16 | 86,0 | 86,0 |

En opérant comme précédemment mais en doublant la durée de chauffage, les rendements réels en dichloro-3,5 aniline sont quantitatifs.

*Exemples 16 et 17:*

On opère comme à l'exemple 1 en remplaçant successivement la tétrachloro-2,3,4,5 aniline par la dichloro-3,4 aniline et la dichloro-2,3 aniline, auxquels cas n est égal à 1, chacune à une concentration de 2 mol/l, l'acide iodhydrique étant à une concentration de 8 mol/l, la température de 135°C et la durée de 5 h.

Dans ces conditions on constate que:

1. La dichloro-3,4 aniline est transformée à 48,4% et le rendement en chloro-3 aniline est de 91,5% par rapport à la dichloro-3,4 aniline transformée;
2. La dichloro-2,3 aniline est transformée à 37,5% et le rendement en chloro-3 aniline est de 88,2% par rapport à la dichloro-2,3 aniline transformée.

*Exemples 18 et 19:*

Dans un réacteur de 200 $cm^3$ de capacité, revêtu intérieurement de tantale et résistant à la pression, on charge:

- $4 \times 10^{-3}$ mol de tétrachloro-2,3,5,6 aniline,
- $16 \times 10^{-3}$ mol d'iodure d'hydrogène liquide,
- 20 ml de trichloro-1,2,4 benzène.

On chauffe sous pression autogène pendant 2 h respectivement à 170 et 210°C.

Au bout de ce temps de réaction, on constate que:

a) à 170°C, le taux de transformation de la tétrachloro-2,3,5,6 aniline est de 63,4%, le rendement réel en dichloro-3,5 aniline est de 57,2% et il reste 6,2% de trichloro-2,3,5 aniline intermédiaire non transformée;

b) à 210°C, le taux de transformation de la tétrachloro-2,3,5,6 aniline est de 84,1%, le rendement réei en dichloro-3,5 aniline est de 80,6% et il reste 3,5% de trichloro-2,3,5 aniline intermédiaire non transformée.

Si l'on répète ces deux essais en chauffant deux fois plus longtemps, on obtient un rendement réel quantitatif en dichloro-3,5 aniline.

*Exemple 20:*

Dans un ballon de verre de 250 $cm^3$, on charge 0,06 mol de tétrachloro-2,3,4,5 aniline et 0,06 mol de tétrachloro-2,3,5,6 aniline avec 100 ml de trichloro-1,2,4-benzène. On agite et on chauffe à 160°C. Lorsque cette température est atteinte, on envoie un courant d'iodure d'hydrogène gazeux sec à raison de 18 à 20 g/h, en maintenant la température à 160°C et sous agitation.

L'effluent gazeux est recueilli dans un barboteur contenant de l'eau.

On stoppe la réaction après 4 h 20 min par arrêt du courant gazeux d'iodure d'hydrogène. On a utilisé 80,5 g de ce dernier.

On soumet alors le mélange à la distillation sous pression atmosphérique en ajoutant progressivement 150 ml de trichloro-1,2,4-benzène. On peut ainsi séparer la presque totalité de l'iode des polychloranilines issues de la réaction.

Le contenu du réacteur est ensuite refroidi, neutralisé avec une solution aqueuse de soude diluée et la phase organique est analysée en chromatographie en phase aqueuse. On constate que le taux de transformation des tétrachloroanilines est de 99,5% et que le rendement en dichloro-3,5 aniline est de 98,1%. Il reste 0,9% de trichloroanilines, intermédiaires de la transformation.

*Exemple 21:*

Dans un ballon tricol de 250 $cm^3$, on charge 100 ml de dichloro-1,2-benzène, 0,168 mol de dichloro-2,3 aniline et 0,072 mol de dichloro-3,4 aniline. On porte à 160°C et on alimente en 4 h 0,66 mol d'iodure d'hydrogène gazeux, soit 2,75 mol HI par mol de DCA, en agitant le milieu réactionnel.

Au bout de ce temps, après refroidissement et mise en œuvre d'un traitement classique (comme décrit dans les exemples précédents), l'analyse en chromatographie en phase vapeur montre que le taux de transformation des dichloroanilines est de 96,5% et le rendement en 3 chloroaniline de 100% par rapport aux dichloroanilines transformées.

L'iode formé est récupéré quantitativement. L'excédent d'iodure d'hydrogène est également récupéré sous forme d'iode, après oxydation par l'eau oxygénée en milieu acide.

**Revendications**

1. Procédé de préparation d'anilines substituées en position méta par du chlore de formule générale:

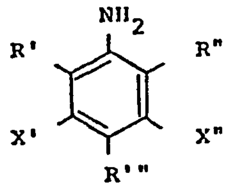

(B)

dans laquelle:

– X' et X'', identiques ou différents, représentent chacun un atome de chlore, un radical alcoyle contenant de 1 à 4 atomes de carbone, un radical alcoxyle contenant de 1 à 4 atomes de carbone, au moins l'un des X' et X'' étant obligatoirement un atome de chlore, l'un des X' et X'' pouvant de plus être un atome d'hydrogène,

– R', R'' et R''', identiques ou différents, représentent chacun un atome de chlore, un radical alcoyle contenant de 1 à 4 atomes de carbone, un radical alcoxyle contenant de 1 à 4 atomes de carbone ou un radical phényle, benzyle ou phénoxyle, le noyau phényle de ces radicaux pouvant être substitué par au moins un atome d'halogène, notamment de chlore, ou un radical alcoyle contenant de 1 à 4 atomes de carbone ou alcoxyle contenant de 1 à 4 atomes de carbone, au plus deux d'entre eux pouvant être un atome d'hydrogène, par réaction d'une aniline polychlorée de formule générale:

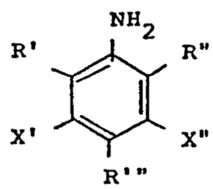

(A)

dans laquelle X', X'', R', R'', R''' ont les mêmes significations que précédemment avec la condition supplémentaire que l'un au moins des R', R'' et R''' représente un atome de chlore, en phase liquide, avec de l'iodure d'hydrogène, selon le schéma:

$$A + 2n\,HI \rightarrow B + n\,I_2 + n\,HCl$$

dans lequel n est un nombre entier de 1 à 3 représentant le nombre d'atomes de chlore à enlever par mole de l'aniline polychlorée A.

2. Procédé selon la revendication 1, caractérisé en ce que le rapport molaire de l'iodure d'hydrogène sur le nombre d'atomes de chlore à enlever par mole de l'aniline polychlorée A de départ est compris entre 2/1 et 20/1.

3. Procédé selon la revendication 2, caractérisé en ce que le rapport molaire de l'iodure d'hydrogène sur le nombre d'atomes de chlore à enlever par mole de l'aniline polychlorée A de départ est compris entre 2/1 et 10/1.

4. Procédé selon l'une des revendications 1 à 3, caractérisé en ce que la réaction est effectuée en milieu aqueux.

5. Procédé selon l'une des revendications 1 à 3, caractérisé en ce que la réaction est effectuée dans un solvant organique inerte.

6. Procédé selon la revendication 5, caractérisé en ce que le solvant organique est un chlorobenzène.

7. Procédé selon l'une des revendications 1 à 4, caractérisé en ce que la réaction est effectuée en présence d'un acide fort.

8. Procédé selon la revendication 7, caractérisé en ce que l'acide fort est l'acide chlorhydrique.

9. Procédé selon l'une des revendications 1 à 8, caractérisé en ce que la réaction est effectuée à une température comprise entre 90 et 250° C, et de préférence entre 110 et 220° C.

10. Procédé selon l'une des revendications 1 à 9, caractérisé en ce que la pression autogène est au plus égale à 100 bars, et de préférence au plus égale à 50 bars.

11. Procédé selon l'une des revendications 1 à 10, caractérisé en ce que dans les formules A et B:

– X' et X'' identiques ou différents représentent un atome de chlore, l'un d'eux pouvant en outre être un atome d'hydrogène,

– R', R'' et R''' identiques ou différents représentent un atome de chlore, au plus deux d'entre eux pouvant de plus être un atome d'hydrogène.

12. Procédé selon la revendication 11, caractérisé en ce qu'on prépare la dichloro-3,5 aniline.

13. Procédé selon la revendication 11, caractérisé en ce qu'on prépare la chloro-3 aniline.

**Patentansprüche**

1. Verfahren zur Herstellung m-chlorsubstituierter Aniline der allgemeinen Formel B

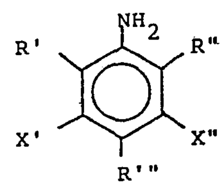

(B),

in der bedeuten:

X' und X'' zugleich oder unabhängig jeweils Chlor, $C_1$- bis $C_4$-Alkyl oder $C_1$- bis $C_4$-Alkoxy, wobei mindestens einer der Substituenten X' und X'' zwingend Chlor ist und ferner einer der Substituenten X' und X'' Wasserstoff bedeuten kann, und

R', R'' und R''' zugleich oder unabhängig jeweils Chlor, $C_1$- bis $C_4$-Alkyl, $C_1$- bis $C_4$-Alkoxy oder Phenyl, Benzyl oder Phenoxy, wobei der Phenylkern dieser Gruppen mit mindestens einem Halogenatom, vorzugsweise Chlor, oder $C_1$- bis $C_4$-Alkyl oder $C_1$- bis $C_4$-Alkoxy substituiert sein kann, wobei ferner höchstens zwei der Substituenten R', R'' und R''' Wasserstoff bedeuten können, gekennzeichnet durch Umsetzung eines polychlorierten Anilins der allgemeinen Formel A

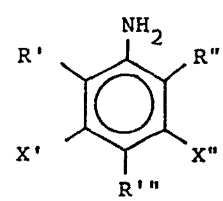

(A),

in der X', X'', R', R'' und R''' die obige Bedeutung besitzen, mit der zusätzlichen Massgabe, dass

mindestens einer der Substituenten R', R'' und R''' Chlor ist, in flüssiger Phase mit Jodwasserstoff nach der Reaktionsgleichung:

$$A + 2n\ HI \rightarrow B + n\ I_2 + n\ HCl$$

worin n eine ganze Zahl von 1 bis 3 ist, die der Anzahl der pro Mol polychloriertes Anilin A zu entfernenden Chloratome entspricht.

2. Verfahrennach Anspruch 1, dadurch gekennzeichnet, dass das Molverhältnis von Jodwasserstoff, bezogen auf die Anzahl der pro Mol des als Ausgangsmaterial eingesetzten polychlorierten Anilins A zu entfernenden Chloratome, zwischen 2:1 und 20:1 liegt.

3. Verfahren nach Anspruch 2, dadurch gekennzeichnet, dass das Molverhältnis von Jodwasserstoff, bezogen auf die Anzahl der pro Mol als Ausgangsmaterial eingesetztem polychlorierten Anilin A zu entfernenden Chloratome, zwischen 2:1 und 10:1 liegt.

4. Verfahren nach einem der Ansprüche 1 bis 3, dadurch gekennzeichnet, dass die Reaktion in wässrigem Medium durchgeführt wird.

5. Verfahren nach einem der Ansprüche 1 bis 3, dadurch gekennzeichnet, dass die Reaktion in einem inerten organischen Lösungsmittel durchgeführt wird.

6. Verfahren nach Anspruch 5, dadurch gekennzeichnet, da die Reaktion in einem Chlorbenzol als Lösungsmittel durchgeführt wird.

7. Verfahren nach einem der Ansprüche 1 bis 4, dadurch gekennzeichnet, dass die Reaktion in Gegenwart einer starken Säure durchgeführt wird.

8. Verfahren nach Anspruch 7, dadurch gekennzeichnet, dass als starke Säure Salzsäure verwendet wird.

9. Verfahren nach einem der Ansprüche 1 bis 8, dadurch gekennzeichnet, dass die Reaktion bei einer Temperatur von 90 bis 250°C, vorzugsweise von 110 bis 220°C, durchgeführt wird.

10. Verfahren nach einem der Ansprüche 1 bis 9, dadurch gekennzeichnet, dass der autogen entstehende Druck höchstens gleich 100 bar, vorzugsweise höchstens gleich 50 bar, ist.

11. Verfahren nach einem der Ansprüche 1 bis 10, dadurch gekennzeichnet, dass solche Verbindungen der Formel A eingesetzt bzw. der Formel B hergestellt werden, worin bedeuten:

X' und X'' zugleich oder unabhängig Chlor, wobei einer dieser Substituenten ferner auch Wasserstoff bedeuten kann, und

R', R'' und R''' zugleich oder unabhängig Chlor, wobei ferner höchstens zwei der Substituenten R', R'' und R''' auch Wasserstoff bedeuten können.

12. Verfahren nach Anspruch 11, dadurch gekennzeichnet, dass 3.5-Dichloranilin hergestellt wird.

13. Verfahren nach Anspruch 11, dadurch gekennzeichnet, dass 3-Chloranilin hergestellt wird.

## Claims

1. A process for the preparation of an aniline substituted in the meta position by chlorine, of the general formula:

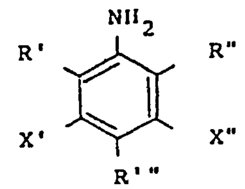

(B)

in which:

– X' and X'', which are identical or different, each represent a chlorine atom, an alkyl radical containing from 1 to 4 carbon atoms or an alkoxy radical containing from 1 to 4 carbon atoms, at least one of the symbols X' and X'' necessarily being a chlorine atom and it also being possible for one of the symbols X' and X'' to be a hydrogen atom, and

– R', R'' and R''', which are identical or different, each represent a chlorine atom, an alkyl radical containing from 1 to 4 carbon atoms, an alkoxy radical containing from 1 to 4 carbon atoms or a phenyl, benzyl or phenoxy radical, it being possible for the phenyl nucleus of these radicals to be substituted by at least one halogen atom, in particular a chlorine atom, an alkyl radical containing from 1 to 4 carbon atoms or an alkoxy radical containing from 1 to 4 carbon atoms, it being possible for at most two of them to be a hydrogen atom, by reacting a polychloroaniline of the general formula:

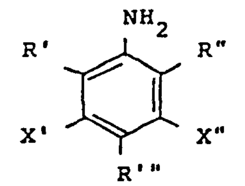

(A)

in which X', X'', R', R'' and R''' have the same meanings as above, with the additional proviso that at least one of the symbols R', R'' and R''' represents a chlorine atom, with hydrogen iodide, in the liquid phase, according to the equation:

$$A + 2n\ HI \rightarrow B + n\ I_2 + n\ HCl$$

in which n is an integer from 1 to 3, representing the number of chlorine atoms to be removed per mol of the polychloroaniline A.

2. The process according to claim 1, wherein the molar ratio of the hydrogen iodide to the number of chlorine atoms to be removed per mol of the starting polychloroaniline A is between 2/1 and 20/1.

3. The process according to claim 2, wherein the molar ratio of the hydrogen iodide to the number of chlorine atoms to be removed per mol of the starting polychloroaniline A is between 2/1 and 10/1.

4. The process according to one of claims 1 to 3, wherein the reaction is carried out in an aqueous medium.

5. The process according to one of claims 1 to 3, wherein the reaction is carried out in an inert organic solvent.

6. The process according to claim 5, wherein the organic solvent is a chlorobenzene.

7. The process according to one of claims 1 to 4, wherein the reaction is carried out in the presence of a strong acid.

8. The process according to claim 7, wherein the strong acid is hydrochloric acid.

9. The process according to one of claims 1 to 8, wherein the reaction is carried out at a temperature of between 90 and 250°C and preferably of between 110 and 220°C.

10. The process according to one of claims 1 to 9, wherein the autogenous pressure is equal to at most 100 bars and preferably equal to at most 50 bars.

11. The process according to one of claims 1 to 10, wherein, in the formulae A and B:

– X′ and X″, which are identical or different, represent a chlorine atom, it also being possible for one of them to be a hydrogen atom, and

– R′, R″ and R‴, which are identical or different, represent a chlorine atom, it also being possible for at most two of them to be a hydrogen atom.

12. The process according to claim 11, wherein 3,5-dichloroaniline is prepared.

13. The process according to claim 11, wherein 3 chloroaniline is prepared.